# EUROPEAN PATENT SPECIFICATION

(11) **EP 2 351 729 B1**
(45) Date of publication and mention of the grant of the patent: **22.01.2014**
(21) Application number: 09823609.4
(22) Date of filing: 28.10.2009
(51) Int. Cl.: C07C 51/43, C07B 57/00, C07C 51/487, C07C 53/134, C07D 311/66

(54) **PROCESS FOR PRODUCTION OF OPTICALLY ACTIVE ORGANIC CARBOXYLIC ACID**
VERFAHREN ZUR HERSTELLUNG VON OPTISCH AKTIVER ORGANISCHER CARBOXYLSÄURE
PROCÉDÉ DE PRODUCTION D'UN ACIDE CARBOXYLIQUE ORGANIQUE OPTIQUEMENT ACTIF

(30) Priority: 29.10.2008 JP 2008278496
(43) Date of publication of application: 03.08.2011
(73) Proprietor: Mitsubishi Gas Chemical Company, Inc., Tokyo 100-8324 (JP)
(72) Inventor: KYUUKO, Youichi, Niigata-shi Niigata 950-3112 (JP)
(74) Representative: Müller-Boré & Partner Patentanwälte PartG mbB
(86) International application number: PCT/JP2009/068484
(87) International publication number: WO 2010/050499

(56) References cited:
- EP-A1- 2 088 144
- WO-A1-02/12221
- WO-A1-2004/108944
- WO-A1-2008/050829
- JP-A- 6 507 159
- JP-A- 7 149 688
- JP-A- 2005 023 055
- JP-A- 2008 044 856
- HIROSHI YAMANAKA: 'Kogaku Kasseitai no Preparation' KOGAKU ISEITAI NO BUNRI [KIKAN KAGAKU SOSETSU] vol. 6, 10 June 1999, pages 2 - 14, XP008146717

## Description

### [Technical Field]

The present invention relates to a technique for improving the enantiomer excess of an optically active organic carboxylic acid. An optically active organic carboxylic acid is highly useful as a raw material for the synthesis of chemical substances required to be chiral, such as pharmaceuticals, agricultural chemicals, and the like.

### [Background Art]

Optically active organic carboxylic acids serve as pharmaceutical intermediates or as important synthetic intermediates of functional chemicals, and the like. Therefore, various methods have been proposed for obtaining optically active forms from a racemate. Examples of such methods for obtaining optically active forms from a racemate include preferential crystallization, a diastereomeric method, an enzymatic method, and chromatography.

Preferential crystallization is a method in which desired crystals are seeded to a supersaturated solution of a racemate, and only such crystals are grown and precipitated. That is, in order to resolve a certain racemate by preferential crystallization, it is neccessary to first measure the solubilities of the racemate and the two optically active forms to confirm that the solubility of the racemate is higher than the solubilities of the two optically active forms; the melting points of the two optically active forms are higher than that of the racemate; and the optically active forms are insoluble in a supersaturated solution of the racemate. It is also necessary to confirm that the infrared absorption spectrum of the racemate matches those of the optically active forms, for example (see, e.g., Nonpatent Document 1).

A diastereomeric method is a method in which an optically active acid or base is allowed to act on a racemate, and perform resolution by fractional crystallization using the solubility of the produced diastereomeric salts (see e.g., Patent Document 1).

An optical resolution method using an enzyme utilizes the stereospecificity of an enzyme to the substrate. It is a method in which, for example, an enzyme of microbial origin with hydrolyzing activity is allowed to act on an ester compound to selectively hydrolyze one optically active form to separate the desired optically active form (see, e.g., Patent Document 2).

A chromatography method using a chiral compound as the stationary phase is a method in which optical resolution is performed by the interaction of the S-form, the R-form, and the filler.

Such resolution does not necessarily achieve sufficient resolution efficiency, and further operations, such as repetitive recrystallization, are sometimes required in order to increase the optical purity of the desired optically active form. The disposal of an unnecessary optically active form resulting from separation may also be a problem. Such an unnecessary optically active form is occasionally racemized and reused. However, because it takes much time and effort to perform separation and purification, such an optically active form is often discarded, abandoning the reuse. Meanwhile, there are also many cases where both the enantiomers are useful, and there has been a demand for a method for, after the removal of one optically active from, efficiently recovering the remaining optically active form. However, generally, the enantiomeric excess of a remaining optically active form is often low, and the efficient recovery thereof takes much time and effort.

### [Prior Art Documents]

### [Patent Documents]

[Patent Document 1] JP-A-2005-23055
[Patent Document 2] WO 2004/108944

### [Nonpatent Documents]

[Nonpatent Document 1] Hiroshi YAMANAKA, Yasuhisa TASHIRO, Kikan Kagaku Sosetsu, No. 6, 1989, pp. 4 to 5

### [Summary of the Invention]

### [Problems that the Invention is to Solve]

An object of the present invention is to provide a simple production method for obtaining an optically active organic carboxylic acid with improved enantiomeric excess.

### [Means for Solving the Problems]

The present inventors conducted extensive research to solve the above problems. As a result, they found the following phenomenon: an optically active organic carboxylic acid with higher enantiomeric excess can be obtained by an extremely simple method, in which a portion of an optically active organic carboxylic acid in the form of an enantiomeric mixture is merely converted to an alkali salt or a free carboxylic acid and separated. They thus accomplished the present invention.

That is, the present invention relates to a method for producing an optically active organic carboxylic acid shown in the following (1) to (7).
(1) A method for producing an optically active organic carboxylic acid, comprising isolating an excess of one enantiomer from an enantiomeric mixture of an optically active organic carboxylic acid where enantiomers are present in a non-equimolar ratio,
   the method including:
   as a first neutralization step, neutralizing the whole quantity of the enantiomeric mixture of an optically active organic carboxylic acid with an alkali to prepare a mixture of enantiomers of an organic carboxylic acid salt;
   as a second neutralization step, adding an acid to the mixture of enantiomers of an organic carboxylic acid salt obtained in the first neutralization step to prepare a resulting organic carboxylic acid and an unneutralized organic carboxylic acid salt, the acid being in an amount of 0.9 to 1.1 times the molar number calculated from twice the molar number of the enantiomer of the optically active organic carboxylic acid being present in a lower molar proportion of enantiomers of the optically active organic carboxylic acid; and
   separating the organic carboxylic acid and unneutralized organic carboxylic acid salt obtained in the second neutralization step from each other, thereby isolating the excess of one enantiomer, which is in the unneutralized organic carboxylic acid salt;
   wherein the optically active organic carboxylic acid is a chroman carboxylic acid represented by the general formula (1) or 2-(4-isobutylphenyl)propionic acid : 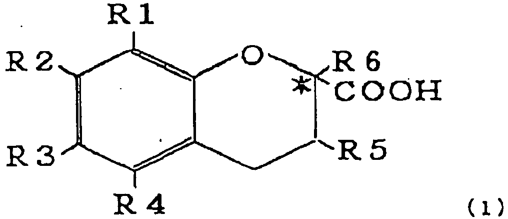
   wherein R1, R2, and R4 to R6 each independently represent a methyl group or a hydrogen atom, R3 represents a hydroxy group, a methoxy group, or a hydrogen atom, and wherein the optically active organic carboxylic acid has an enantiomeric excess of 2 to 98% ee.
(2) A method for producing an optically active organic carboxylic acid, comprising isolating an excess of one enantiomer from an enantiomeric mixture of an optically active organic carboxylic acid where enantiomers are present in a non-equimolar ratio,
   the method including:
   adding an alkali to the enantiomeric mixture of an optically active organic carboxylic acid to prepare a resulting organic carboxylic acid salt and an unneutralized organic carboxylic acid, the alkali being in an amount of 0.9 to 1.1 times the molar number calculated from [the total molar number of the optically active organic carboxylic acid - (twice the molar number of the enantiomer of the optically active organic carboxylic acid being present in a lower molar proportion of enantiomers of the optically active organic carboxylic acid)]; and
   separating the obtained organic carboxylic acid salt and unneutralized organic carboxylic acid from each other, thereby isolating the excess of one enantiomer, which is in the organic carboxylic acid salt;
   wherein the optically active organic carboxylic acid is a chroman carboxylic acid represented by the general formula (1) or 2-(4-isobutylphenyl)propionic acid : 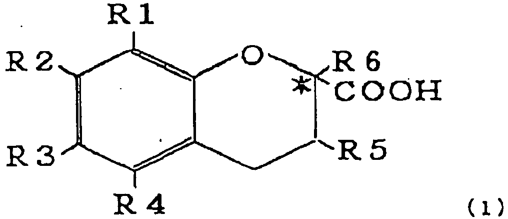 wherein R1, R2, and R4 to R6 each independently represent a methyl group or a hydrogen atom, R3 represents a hydroxy group, a methoxy group, or a hydrogen atom, and wherein the optically active organic carboxylic acid has an enantiomeric excess of 2 to 98% ee.
(3) A method for producing an optically active organic carboxylic acid according to (1) or (2), wherein a mixture of equal amounts of enantiomers of the optically active organic carboxylic acid has a melting point higher than the melting points of both the S-form and R-form enantiomers of the optically active organic carboxylic acid.
(4) A method for producing an optically active organic carboxylic acid according to any one of (1) to (3), wherein the optically active organic carboxylic acid is 6-hydroxy-2,5,7,8-tetramethylchroman-2-carboxylic acid.
(5) A method for producing an optically active organic carboxylic acid according to any one of (1) to (3), wherein the optically active organic carboxylic acid is 2-(4-isobutylphenyl)propionic acid.
(6) A method for producing an optically active organic carboxylic acid according to (1), wherein an inorganic acid or an optically inactive organic acid is used as the acid for neutralization.
(7) A method for producing an optically active organic carboxylic acid according to (1) or (2), wherein a hydroxide or carbonate of an alkali metal, ammonia, or an optically inactive organic amine is used as the alkali for neutralization.

### [Advantage of the Invention]

According to the present invention, the enantiomeric excess of an optically active organic carboxylic acid which is useful as a pharmaceutical raw material or synthetic intermediate can be efficiently increased by a simple method.

### [Mode for Carrying Out the Invention]

As methods for producing an optically active organic carboxylic acid according to the present invention, which comprise isolating an excess of one enantiomer, the following first method and second method are mentioned.

That is, the first method of the present invention is a method for isolating an excess of one enantiomer from an enantiomeric mixture of an optically active organic carboxylic acid where enantiomers are present in a non-equimolar ratio. The method includes, as a first neutralization step, neutralizing the whole quantity of the enantiomeric mixture of an optically active organic carboxylic acid with an alkali to prepare a mixture of enantiomers of an organic carboxylic acid salt; as a second neutralization step, adding an acid to the mixture of enantiomers of an organic carboxylic acid salt obtained in the first neutralization step to prepare a resulting organic carboxylic acid and an unneutralized organic carboxylic acid salt, the acid being in an amount of 0.9 to 1.1 times the molar number calculated from twice the molar number of, of the enantiomers, the enantiomer present in a lower molar proportion; and separating the organic carboxylic acid and unneutralized organic carboxylic acid salt obtained in the second neutralization step from each other, thereby isolating the excess of one enantiomer, which is in the unneutralized organic carboxylic acid salt.

The second method of the present invention is a method for isolating an excess of one enantiomer from an enantiomeric mixture of an optically active organic carboxylic acid where enantiomers are present in a non-equimolar ratio. The method includes adding an alkali to the enantiomeric mixture of an optically active organic carboxylic acid to prepare a resulting organic carboxylic acid salt and an unneutralized organic carboxylic acid, the alkali being in an amount of 0.9 to 1.1 times the molar number calculated from [the total molar number of the optically active organic carboxylic acid - (twice the molar number of, of enantiomers of the optically active organic carboxylic acid, the enantiomer present in a lower molar proportion)]; and separating the obtained organic carboxylic acid salt and unneutralized organic carboxylic acid from each other, thereby isolating the excess of one enantiomer, which is in the organic carboxylic acid salt.

In the implementation of the present invention, in terms of the optical purity and yield of the desired optically active organic carboxylic acid, it is necessary to predetermine the amount of alkali or acid required for giving an alkali salt of the organic carboxylic acid or an educt of the organic carboxylic acid. For this reason, in order to specify the enantiomeric ratio of the organic carboxylic acid, it is necessary to measure the amount of each enantiomer using a known analysis technique, such as high-speed liquid chromatography (HPLC) or gas chromatography.

The optically active organic carboxylic acid used in the present invention may be a chroman carboxylic acid represented by the general formula (1) or 2-(4-isobutylphenyl)propionic acid.

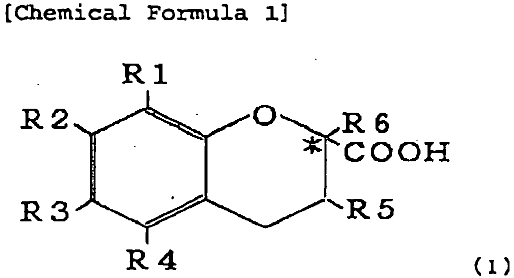

In the general formula (1), R1, R2, and R4 to R6 each independently represent a methyl group or a hydrogen atom. R3 represents a hydroxy group, a methoxy group, or a hydrogen atom.

The mixture of such an optically active organic carboxylic acid used in the present invention is an enantiomeric mixture in which the enantiomers are present in a non-equimolar ratio. The method of the present invention is applicable to the case where the ratio between the two enantiomers of the optically active organic carboxylic acid is not equimolar. That is, the optically active organic carboxylic acid preferably has an enantiomeric excess of 2 to 98% ee, more preferably 5 to 95% ee, and still more preferably 10 to 90% ee.

The term "enantiomeric excess" indicates the optical purity of a chiral compound, and is abbreviated to ee. The ee is expressed as a value obtained by subtracting the amount of the minor substance from the amount of the major substance, and dividing the difference by the total amount of substances (it is also possible to subtract the proportion of the minor substance from the proportion of the major substance).

Further, with respect to the properties of the desired organic carboxylic acid, when the racemate has a higher melting point than the enantiomers (S-form and R-form), the present invention can be expected to be more effective. From this point of view, with respect to the melting point of the organic carboxylic acid, it is preferable that the melting point of the racemate is 5°C or more higher, more preferably 10°C or more higher, still more preferably 20°C or more higher, than the melting points of the enantiomers (S-form and R-form). A racemate refers to a 50:50 mixture of equal amounts of the S-form and the R-form.

Specific examples of compounds with melting points of such characteristics include, but are not limited to, 6-hydroxy-2,5,7,8-tetramethylchroman-2-carboxylic acid and 2-(4-isobutylphenyl)propionic acid.

An enantiomeric mixture of the optically active organic carboxylic acid where enantiomers are present in a non-equimolar ratio, which is to be used as a raw material in the present invention, may be a method in which one of the enantiomers is removed by stereoselectively esterifying the optically active organic carboxylic acid or by using an optical resolution agent, for example.

The esterification method may be as follows, for example. The optically active organic carboxylic acid is stereoselectively esterified in an organic solvent in the presence of an alcohol using a biocatalyst, then an alkali is added thereto in the presence of an organic solvent that causes layer isolation with water, and an optically active organic carboxylic acid salt in the aqueous layer is separated from an optically active organic carboxylic acid ester in the organic layer.

As the biocatalyst, a hydrolase produced by a microorganism, such as a stereoselective lipase of microbial origin, or a modification thereof can be used. Lipases from microorganisms of the genus Candida and modifications thereof are preferable. In particular, a lipase produced by Candida antarctica allows the esterification reaction to proceed smoothly, and thus is particularly preferable for high enantioselectivity, yield, etc. The form of the biocatalyst is not limited, and it may be used as an enzyme directly or as an immobilized enzyme. It is also possible to directly use microbial cells as a catalyst.

As alcohols and solvents for use in the esterification reaction, those described in Patent Document 2 can be mentioned. Further, the reaction conditions for the esterification reaction are the same as the reaction conditions described in Patent Document 2.

In the above method, only one enantiomer in the enantiomeric mixture is esterified by the action of the biocatalyst, and the other enantiomer is left unreacted. Therefore, by the above method, either the S-form enantiomer or the R-form enantiomer is selectively converted to an optically active ester, which is then separated, whereby an enantiomeric mixture of an optically active organic carboxylic acid where enantiomers are present in a non-equimolar ratio can be produced.

The method that uses an optical resolution agent may be as follows. An optically active optical resolution agent is allowed to react with a racemate to produce diastereomers, then the diastereomers are separated using their difference in physical properties, and the diastereomers are decomposed, whereby a non-equimolar enantiomeric mixture can be obtained.

The optical resolution agent is preferably an optically active organic amine, and more preferably optically active N-benzyl-α-phenyl ethylamine. Various approaches can be applied to the separation and purification of diastereomers, such as column chromatography and fractional crystallization.

In the present invention, in separating a relative excess of an optically active organic carboxylic acid from an enantiomeric mixture of the optically active organic carboxylic acid where enantiomers are present in a non-equimolar ratio, it is particularly important, as mentioned above, to accurately analyze the amounts of the two enantiomers to be subjected to separation contained in the enantiomeric mixture of the optically active organic carboxylic acid, and to accurately determine the amount of alkali or acid to be used for neutralization.

In the first method of the present invention, the amount of alkali for use in the first neutralization step is preferably 0.9 to 1.1 times, more preferably 0.95 to 1.05 times, the total molar number of the optically active organic carboxylic acid. In order to recover the desired optically active organic carboxylic acid as intended, it is further preferable to adjust the amount to be 1.00 time the total molar number if possible.

In the first method of the present invention, the amount of acid for use in the second neutralization step is 0.9 to 1.1 times, preferably 0.95 to 1.05 times, the molar number calculated from twice the molar number of, of enantiomers of the optically active organic carboxylic acid, the enantiomer present in a lower molar proportion. In order to recover the desired optically active organic carboxylic acid as intended, it is further preferable to adjust the amount to be 1.00 time the total molar number if possible. In the present invention, "twice the molar number of, of enantiomers of the optically active organic carboxylic acid, the enantiomer present in a lower molar proportion" can also be calculated, using the enantiomeric excess (ee) of the optically active organic carboxylic acid for use in the present invention, by the equation [the total molar number of the optically active organic carboxylic acid × (100 - ee)/100].

In the second neutralization step, when the amount of acid added is controlled to be within the above range, in one enantiomer of the optically active organic carboxylic acid salt obtained in the first neutralization step, which is present in a higher molar proportion, an excess relative to the enantiomer present in a lower molar proportion is left unneutralized and exists as a carboxylic acid alkali salt.

In the two enantiomers to be subjected to separation, when the S-form is in excess of the R-form, the amount of acid used for neutralization is 0.9 to 1.1 times, preferably 0.95 to 1. 05 times, the molar number calculated from twice the molar number of the R-form. When the amount of acid used for neutralization is within the above range, the enantiomeric excess of the S-form can be efficiently increased. Conversely, when the R-form to be separated is in excess of the S-form, the enantiomeric excess of the R-form can be increased efficiently. When the amount of acid used is more than 1.1 times the molar number, the enantiomeric excess does not sufficiently increase. Meanwhile, when the amount of acid used is less than 0.9 times the molar number, although the resulting optically active organic carboxylic acid has improved enantiomeric excess, the yield thereof decreases significantly.

In the second method of the present invention, the amount of alkali for use in the neutralization step is 0.9 to 1.1 times, preferably 0.95 to 1. 05 times, the molar number calculated from [the total molar number of the optically active organic carboxylic acid - (twice the molar number of, of enantiomers of the optically active organic carboxylic acid, the enantiomer present in a lower molar proportion)]. In order to recover the desired optically active organic carboxylic acid as intended, it is further preferable to adjust the amount to be 1.00 time the total molar number if possible.

When the amount of alkali used for neutralization is controlled to be within the above range, in one enantiomer of the optically active organic carboxylic acid, which is present in a higher molar proportion, an excess relative to the enantiomer present in a lower molar proportion is neutralized and exists as a carboxylic acid alkali salt.

In the two enantiomers to be subjected to separation, in the case where the S-form is in excess of the R-form, when the amount of alkali used for neutralization is within the above range, the enantiomeric excess of the S-form can be efficiently increased. Conversely, when the R-form to be separated is in excess of the S-form, the enantiomeric excess of the R-form can be increased efficiently. When the amount of alkali used is more than 1.1 times the molar number, the enantiomeric excess does not sufficiently increase. Meanwhile, when the amount of alkali used is less than 0.9 times the molar number, although the resulting optically active organic carboxylic acid has improved enantiomeric excess, the yield thereof decreases significantly.

Examples of alkalis for neutralization for derivatizing an organic carboxylic acid to a salt for use in the present invention include alkali metal hydroxides, alkali metal carbonates, ammonia, and optically inactive organic amines. Specifically, examples of alkali metal hydroxides include sodium hydroxide, potassium hydroxide, cesium hydroxide, and rubidium hydroxide, and examples of alkali metal carbonates include sodium carbonate, potassium carbonate, cesium carbonate, and rubidium carbonate. Examples of optically inactive organic amines include methylamine, dimethylamine, trimethylamine, ethylamine, diethylamine, and triethylamine. Alkalis for neutralization are not limited to these. However, this method is different from the diastereomeric resolution method, and thus there is no need to use an optically active amine as in diastereomeric resolution.

Examples of acids used for neutralizing an organic carboxylic acid salt include inorganic acids such as sulfuric acid, hydrochloric acid, nitric acid, potassium hydrogen sulfate, and sodium hydrogen sulfate and optically inactive organic acids such as formic acid, acetic acid, propionic acid, and butanoic acid. Acids are not limited to these, and those that convert an organic carboxylic acid salt to a free carboxylic acid are usable.

It is preferable to perform such neutralization in an aqueous solution, but it is also possible to perform it in a mixed solvent of water and a water-miscible solvent. Examples of such water-miscible solvents include methanol, ethanol, 2-propanol, and like alcohols, acetonitrile, and dimethylformamide. Solvents are not limited to these, and can be arbitrarily selected according to the solubility of a salt, the solubility of a substrate, and the like.

The ratio of the substrate containing an organic carboxylic acid salt and an organic carboxylic acid to the solvent can be arbitrarily determined. In actual operation, it is preferable to determine the ratio considering the properties of a substance to be treated. For example, when an alkali salt of the organic carboxylic acid does not sufficiently dissolve in a solution, the proportion of the solvent can be increased. Meanwhile, an extreme increase in the amount of solvent is followed by a decrease in economical efficiency or an increase in environmental burden, and can thus be said to be undesirable. In any case, in industrial implementation, it is preferable to optimize them prior to implementation.

The temperature for the formation of an organic carboxylic acid salt can be arbitrarily determined, and is preferably 0 to 100°C in terms of operativity. In implementation, the temperature can be determined considering the physical properties of the substrate, etc. Further, the temperature for the neutralization of an organic carboxylic acid salt can be arbitrarily determined, and is preferably 0 to 100°C in terms of operativity. In implementation, the temperature can be determined by considering the physical properties of the substrate, etc. Operationally, the temperature for separation is preferably near room temperature, but can be determined considering the physical properties of the substrate, etc., in implementation.

Although it is possible to add an acid and an alkali at once, it is more preferable to add them little by little. Further, although it is possible to perform separation immediately after the addition, stirring for about 1 hour can further enhance the desired enantiomeric-excess-improving effects. Heating may also further enhance the effects.

The organic carboxylic acid and organic carboxylic acid salt obtained above are each separated, and the organic carboxylic acid salt is converted to a free organic carboxylic acid by the addition of an acid, and thus recovered. When such a free organic carboxylic acid is recovered, in the case where the carboxylic acid is in the form of crystals, it can be easily separated by filtration. Examples of separation methods include vacuum filtration, pressure filtration, and centrifugal filtration. Separation methods are not limited to these, and, in industrial implementation, an efficient separation technique can be suitably selected considering the form of crystals, etc. When the organic carboxylic acid is liquid and has good layer isolation properties with water or an aqueous solution, it can be separated directly by decantation, etc. When the organic carboxylic acid is soluble in a solution, it is preferable to perform extraction using a solvent immiscible with water, such as ethyl acetate, toluene, and ethyl ether. When the organic carboxylic acid solution recovered by extraction is concentrated to dryness, an organic carboxylic acid with improved enantiomer percentage can be easily recovered.

As described above, use of the method of the present invention makes it possible to efficiently convert a carboxylic acid with a low enantiomeric excess to a carboxylic acid with a high enantiomeric excess.

### [Examples]

Hereinafter, the present invention will be described in further detail with reference to Examples and Comparative Examples. However, the present invention is not limited to these examples.

### Production Example 1

### Asymmetric esterification of 6-hydroxy-2,5,7,8-tetramethylchroman-2-carboxylic acid

100 g (399.5 mmol) of 6-hydroxy-2,5,7,8-tetramethylchroman-2-carboxylic acid as a raw material, 60 g of methanol, 1500 g of isopropyl ether, and 40 g of an immobilized enzyme Novozym 435 (manufactured by NOVOZYMES) as an asymmetric esterification catalyst were placed in a 5-L pressure-resistant vessel made of stainless steel. The vessel was flushed with argon, and then a reaction was effected at 80°C for 24 hours. After 24 hours, the catalyst was removed by filtration, and the reaction mixture was recovered. As a result of an analysis of the reaction mixture, the conversion of the raw material 6-hydroxy-2,5,7,8-tetramethylchroman-2-carboxylic acid was 45.0 mol%, and the optical purity of methyl S-(-)-6-hydroxy-2,5,7,8-tetramethylchroman-2-carboxylate was 99% ee. Meanwhile, the optical purity of R-(+)-6-hydroxy-2,5,7,8-tetramethylchroman-2-carboxylic acid was 81% ee. The analysis of optical purity was performed by high-speed liquid chromatography (HPLC). The reaction mixture thus obtained was equally divided into 10 portions, and used in the following tests.

### Example 1

### (1) Recovery of unreacted 6-hydroxy-2,5,7,8-tetramethylchroman-2-carboxylic acid

To the reaction mixture equally divided into 10 portions was added 219.7 ml of 0.1 N sodium hydroxide (399.5 mmol/10 × (1 - 0.45) = 21.97 mmol). Unreacted 6-hydroxy-2,5,7,8-tetramethylchroman-2-carboxylic acid was thus converted into a sodium salt and removed in the aqueous layer, followed by recovery.

### (2) Recovery of R-(+)-6-hydroxy-2,5,7,8-tetramethylchroman-2-carboxylic acid

The aqueous layer recovered in (1) above was placed in a 500-mL glass vessel. While heating to 50°C with stirring, 4.17 ml out of 21.97 ml (21.97 mmol) of 1 N hydrochloric acid prepared (21.97 mmol × (100 - 81)/100 = 4.17 mmol) was added thereto, and free primary crystals were removed by filtration. Next, the remaining 17.80 ml of 1 N hydrochloric acid was added to the obtained filtrate, and free secondary crystals were removed by filtration.

The yield of the primary crystals was 0.95 g, and the optical purity of R-(+)-6-hydroxy-2,5,7,8-tetramethylchroman-2-carboxylic acid in the crystals was 7.4% ee.

The yield of the secondary crystals was 4.50 g, and the optical purity of R-(+)-6-hydroxy-2,5,7,8-tetramethylchroman-2-carboxylic acid in the crystals was 97.3% ee. The results are shown in Table 1.

### Example 2

The aqueous layer recovered in (1) in Example 1 was placed in a 500-mL glass vessel. While heating to 50°C with stirring, 4.05 ml out of 21.97 ml (21.97 mmol) of 1 N hydrochloric acid prepared (21.97 ml × (100 - 81)/100 × 0.97 times) was added thereto, and free primary crystals were removed by filtration. Subsequently, the remaining 17.92 ml of 1 N hydrochloric acid was added to the obtained filtrate, and free secondary crystals were removed by filtration.

The yield of the primary crystals was 0.80 g, and the optical purity of R-(+)-6-hydroxy-2,5,7,8-tetramethylchroman-2-carboxylic acid in the crystals was 2.7% ee.

The yield of the secondary crystals was 4.63 g, and the optical purity of R-(+)-6-hydroxy-2,5,7,8-tetramethylchroman-2-carboxylic acid in the crystals was 95.4% ee. The results are shown in Table 1.

### Example 3

The aqueous layer recovered in (1) in Example 1 was placed in a 500-mL glass vessel. While heating to 50°C with stirring, 4.30 ml out of 21.97 ml (21.97 mmol) of 1 N hydrochloric acid prepared (21.97 ml × (100 - 81)/100 × 1.03 times) was added thereto, and free primary crystals were removed by filtration. Subsequently the remaining 17.67 ml of 1 N hydrochloric acid was added to the obtained filtrate, and free secondary crystals were removed by filtration.

The yield of the primary crystals was 1.00 g, and the optical purity of R-(+)-6-hydroxy-2,5,7,8-tetramethylchroman-2-carboxylic acid in the crystals was 8.3% ee.

The yield of the secondary crystals was 4.40 g, and the optical purity of R-(+)-6-hydroxy-2,5,7,8-tetramethylchroman-2-carboxylic acid in the crystals was 98.0% ee. The results are shown in Table 1.

### Comparative Example 1

The aqueous layer recovered in (1) in Example 1 was placed in a 500-mL glass vessel. While heating to 50°C with stirring, 0.88 ml out of 21.97 ml (21.97 mmol) of 1 N hydrochloric acid prepared (21.97 ml × (100 - 81)/100 × 0.21 times) was added thereto, and free primary crystals were removed by filtration. Subsequently the remaining 21.09 ml of 1 N hydrochloric acid was added to the obtained filtrate, and free secondary crystals were removed by filtration.

The yield of the primary crystals was 0.21 g, and the optical purity of R-(+)-6-hydroxy-2,5,7,8-tetramethylchroman-2-carboxylic acid in the crystals was 2.1% ee.

The yield of the secondary crystals was 5.20 g, and the optical purity of R-(+)-6-hydroxy-2,5,7,8-tetramethylchroman-2-carboxylic acid in the crystals was 84.3% ee. The results are shown in Table 1.

### Comparative Example 2

The aqueous layer recovered in (1) in Example 1 was placed in a 500-mL glass vessel. While heating to 50°C with stirring, 6.39 ml out of 21.97 ml (21.97 mmol) of 1 N hydrochloric acid prepared (21.97 ml × (100 - 81)/100 × 1.53 times) was added thereto, and free primary crystals were removed by filtration-Next, the remaining 15.58 ml of 1 N hydrochloric acid was added to the obtained filtrate, and free secondary crystals were removed by filtration.

The yield of the primary crystals was 1.50 g, and the optical purity of R-(+)-6-hydroxy-2,5,7,8-tetramethylchroman-2-carboxylic acid in the crystals was 37.5% ee.

The yield of the secondary crystals was 3.88 g, and the optical purity of R-(+)-6-hydroxy-2,5,7,8-tetramethylchroman-2-carboxylic acid in the crystals was 97.9% ee. The results are shown in Table 1.

[Table 1]

**Table 1. Change In Enantiomeric Excess upon Neutralization of Sodium Salt with Hydrochloric Acid (Neutralization at 50°C)**

| | | Amount of Acid Added (Primary Crystals) | | | Amount of Acid Added (secondary Crystals) | | |
|---|---|---|---|---|---|---|---|
| Examples | Ratio to Theory | mmol | Yield | Optical Purity | mmol | Yield | Optical purity |
| Raw Material Liquid | | | | | | 5.50 g | 81.0% ee |
| Example 1 | 1.00 | 4.17 | 0.95 g | 7.4% ee | 17.80 | 4.50 g | 97.3% ee |
| Example 2 | 0.97 | 4.05 | 0.80 g | 2.7% ee | 17.92 | 4.63 g | 95.4% ee |
| Example 3 | 1.03 | 4.30 | 1.00 g | 8.3% ee | 17.67 | 4.40 g | 98.0% ee |
| Comparative Example 1 | 0.21 | 0.88 | 0.21 g | 2,1% ee | 21.09 | 5.02 g | 84.3% ee |
| Comparative Example 2 | 1.53 | 6.39 | 1.50 g | 37.5% ee | 15.58 | 3.88 g | 97.9% ee |

### Example 4

### (1) Recovery of unreacted 6-hydroxy-2,5,7,8-tetramethylchroman-2-carboxylic acid

To the reaction mixture obtained in Production Example 1 equally divided into 10 portions was added 21.97 ml of 0.1 N ammonia (39.95 × (1 - 0.45) = 21.97 mmol). Unreacted 6-hydroxy-2,5,7,8-tetramethylchroman-2-carboxylic acid was thus converted into an ammonium salt and removed in the aqueous layer, followed by recovery.

### (2) Recovery of R-(+)-6-hydroxy-2,5,7,A-tetramethylchroman-2-carboxylic acid

21.97 ml (21.97 mmol) of 1 N hydrochloric acid was prepared. Subsequently, the aqueous layer recovered above was placed in a 500-mL glass vessel, and 4.17 ml of 1 N hydrochloric acid (21. 97 ml × (100 - 81)/100) was added thereto while heating to 50°C with stirring. After free primary crystals were removed by filtration, the remaining 17.8 ml of 1 N hydrochloric acid was added thereto, and free secondary crystals were collected by filtration.

The yield of the primary crystals was 0.94 g, and the optical purity of R-(+)-6-hydroxy-2,5,7,8-tetramethylchroman-2-carboxylic acid in the primary crystals was 7.1% ee.

The yield of the secondary crystals was 4.51 g, and the optical purity of R-(+)-6-hydroxy-2,5,7,8-tetramethylchroman-2-carboxylic acid in the secondary crystals was 97.2% ee. The results are shown in Table 2.

### Example 5

21.97 ml (21.97 mmol) of 1 N hydrochloric acid was prepared, and the aqueous layer recovered in (1) in Example 1 was placed in a 500-mL glass vessel, and 4.13 ml of 1 N hydrochloric acid (21.97 ml × (100 - 81)/100 × 0.99 times) was added thereto while heating to 50°C with stirring. After free primary crystals were removed by filtration, the remaining 17.82 ml of 1 N hydrochloric acid was added thereto. Free secondary crystals were collected by filtration.

The yield of the primary crystals was 0.95 g, and the optical purity of R-(+)-6-hydroxy-2,5,7,8-tetramethylchroman-2-carboxylic acid in the primary crystals was 8.3% ee.

The yield of the secondary crystals was 4.40 g, and the optical purity of R-(+)-6-hydroxy-2,5,7,8-tetramethylchroman-2-carboxylic acid in the secondary crystals was 96.7% ee. The results are shown in Table 2.

### Comparative Example 3

21.97 ml (21.97 mmol) of 1 N hydrochloric acid was prepared, and the aqueous layer recovered in (1) in Example 1 and 50 ml of methanol were placed in a 500-mL glass vessel, and 6.37 ml of 1 N hydrochloric acid was added thereto while heating to 50°C with stirring. After free primary crystals were removed by filtration, the remaining 15.60 ml of 1 N hydrochloric acid was added thereto. Free secondary crystals were collected by filtration.

The yield of the primary crystals was 1.51 g, and the optical purity of R-(+)-6-hydroxy-2,5,7,8-tetramethylchroman-2-carboxylic acid in the primary crystals was 37.5% ee.

The yield of the secondary crystals was 3.87 g, and the optical purity of R-(+)-6-hydroxy-2,5,7,8-tetramethylchroman-2-carboxylic acid in the secondary crystals was 97.9% ee. The results are shown in Table 2.

[Table 2]

**Table 2. Change in Enantiomeric Excess upon Neutralization of Ammonium Salt with Hydrochloric Acid (Neutralization at 50°C)**

| | | Amount of Acid Added (Primary Crystals) | | | Amount of Acid Added (Secondary Crystals) | | |
|---|---|---|---|---|---|---|---|
| Examples | Ratio to Theory | mmol | Yield | Optical Purity | mmol | Yield | Optical Purity |
| Raw Material Liquid | | | | | | 5.50 g | 81.0% ee |
| Example 4 | 1.00 | 4.17 | 0.94 g | 7.1% ee | 17.80 | 4.51 g | 97.2% ee |
| Example 5 | 0.99 | 4.13 | 0.95 g | 8.3% ee | 17.82 | 4.40 g | 96.7% ee |
| Comparative Example 3 | 1.53 | 6.37 | 1.51 g | 37.5% ee | 15.60 | 3.87 g | 97.9% ee |

### Example 6

6.40 g (31.02 mmol) of R-2- (4-isobutylphenyl)propionic acid with an optical purity of 50% ee and 310.2 ml (31.02 mmol) of 0.1 N sodium hydroxide were placed in a 500-mL glass vessel, and dissolved. 31. 02 ml (31.02 mmol) of 1 N hydrochloric acid was prepared, and, while stirring at 25°C, 15.51 ml of 1 N hydrochloric acid (31.02 ml × (100 - 50) /100) was added thereto. After free primary crystals were removed by filtration, the remaining 15.5 ml of 1 N hydrochloric acid was added thereto, and free secondary crystals were collected by filtration.

The yield of the primary crystals was 3.20 g, and the optical purity of R-2- (4-isobutylphenyl) propionic acid in the primary crystals was 14% ee.

The yield of the secondary crystals was 3.10 g, and the optical purity of R-2- (4-isobutylphenyl)propionic acid in the secondary crystals was 86% ee. The results are shown in Table 3.

### Example 7

21.97 ml (21.97 mmol) of 1 N hydrochloric acid was prepared. The aqueous layer recovered in (1) in Example 1 was placed in a 500-mL glass vessel, and 4.09 ml of 1 N hydrochloric acid (21.97 ml × (100 - 81) /100 × 0.98 times) was added thereto while heating to 70°C with stirring. After free primary crystals were removed by filtration, 17.87 ml of 1 N hydrochloric acid was added thereto. Free secondary crystals were collected by filtration.

The yield of the primary crystals was 0.85 g, and the optical purity of R-(+)-6-hydroxy-2,5,7,8-tetramethylchroman-2-carboxylic acid in the primary crystals was 3.5% ee.

The yield of the secondary crystals was 4.57 g, and the optical purity of R-(+)-6-hydroxy-2,5,7,8-tetramethylchroman-2-carboxylic acid in the secondary crystals was 95.8% ee. The results are shown in Table 3.

### Example 8

6.00 g (23.97 mmol) of 6-hydroxy-2,5,7,8-tetramethylchroman-2-carboxylic acid with an optical purity of 50% ee and 239.7 ml (11.98 mmol) of 0.05 N sodium hydroxide were placed in a 500-mL glass vessel, and heated to 50°C with stirring for 1 hour. After 1 hour, undissolved crystals were removed by filtration, and then 11.98 ml of 1 N hydrochloric acid was added thereto. Free secondary crystals were collected by filtration.

The yield of the primary crystals was 2.92 g, and the optical purity of the primary crystals was 5.2% ee.

The yield of the secondary crystals was 2-85 g, and the optical purity of the secondary crystals was 94.2%- ee. The results are shown in Table 3.

[Table 3]

**Table 3. Change in Enantiomeric Excess upon Neutralization of Ammonium Salt with Hydrochloric Acid (Neutralization at 70°C)**

| | | Amount of Acid Added (Primary Crystals) | | | Amount of Acid Added (Secondary Crystals) | | |
|---|---|---|---|---|---|---|---|
| Example | Ratio to Theory | mmol | Yield | Optical Purity | mmol | Yield | Optical Purity |
| Raw Material Liquid | | | | | | 5.50 g | 81.0% ee |
| Example 7 | 0.98 | 4.09 | 0.85 g | 3.5% ee | 17.87 | 4.57 g | 95.8% ee |
| Example 8 | 0.99 | 4.13 | 2.92 g | 5.2% ee | 17.82 | 2.85 g | 94.2% ee |

### Production Example 2

### Diastereomeric resolution of 6-hydroxy-2,5,7,8-tetramethylchroman-2-carboxylic acid

10.00 g (39.95 mmol) of 6-hydroxy-2,5,7,8-tetramethylchroman-2-carboxylic acid, 8.45 g (39.95 mmol) of R- (+)-N-benzyl-α-phenylethylamine, and 100 ml of isopropanol were mixed, heated to 50°C, and then left to stand overnight at a temperature of 2°C. The precipitated crystals were filtered, thereby recovering crystals (dry weight: 5.17 g, recovery: 28%) and a mother liquor.

To the 5.17 g of crystals thus obtained, 12 ml of a 1 N aqueous sodium hydroxide solution was added for dissolution. The solution was extracted twice with 50 ml of methyl tertiary butyl ether (MTBE) and washed, and then 12 ml of 1 N hydrochloric acid was added for neutralization. The precipitated crystals were filtered, dried, and analyzed. As a result, the yield of the crystals was 2.75 g, and the optical purity of R-(+)-6-hydroxy-2,5,7,8-tetramethylchroman-2-carboxylic acid was 98 ee%.

### Example 9

### Production of S-(-)-6-hydroxy-2,5,7,8-tetramethylchroman-2-carboxylic acid

The mother liquor (38.1% ee) obtained upon diastereomeric resolution in Production Example 2 was evaporated and dried. 287.6 ml of a 0.1 N aqueous sodium hydroxide solution (39.95 × (1 - 0.28) = 28.76 mmol) was then added thereto, and extracted twice with 50 ml of MTBE and washed. 28.76 ml of 1 N hydrochloric acid was prepared, and 17.83 ml of 1 N hydrochloric acid was first added to the aqueous layer recovered as above while heating to 50°C with stirring. After primary crystals were collected by filtration, 10.93 ml of 1 N hydrochloric acid was added thereto, and secondary crystals were collected by filtration.

The yield of the primary crystals was 4.35 g, and the optical purity of S-(-)-6-hydroxy-2,5,7,8-tetramethylchroman-2-carboxylic acid in the crystals was 2.3% ee.

The yield of the secondary crystals was 2.70 g, and the optical purity of S-(-)-6-hydroxy-2,5,7,8-tetramethylchroman-2-carboxylic acid in the crystals was 94.6% ee.

### [Industrial applicability]

The present invention makes it possible to provide a simple production method for obtaining an optically active organic carboxylic acid with improved enantiomeric excess from an enantiomeric mixture of an optically active organic carboxylic acid where enantiomers are present in a non-equimolar ratio. An optically active organic carboxylic acid with improved enantiomeric excess is highly useful as a raw material for the synthesis of chemical substances required to be chiral, such as pharmaceuticals, agricultural chemicals, and the like.

## Claims

1. A method for producing an optically active organic carboxylic acid, comprising isolating an excess of one enantiomer from an enantiomeric mixture of an optically active organic carboxylic acid where enantiomers are present in a non-equimolar ratio,
the method including:
as a first neutralization step, neutralizing the whole quantity of the enantiomeric mixture of the optically active organic carboxylic acid with an alkali to prepare a mixture of enantiomers of an organic carboxylic acid salt;
as a second neutralization step, adding an acid to the mixture of enantiomers of an organic carboxylic acid salt obtained in the first neutralization step to prepare a resulting organic carboxylic acid and an unneutralized organic carboxylic acid salt, the acid being in an amount of 0.9 to 1.1 times the molar number calculated from twice the molar number of the enantiomer of the optically active organic carboxylic acid being present in a lower molar proportion of enantiomers of the optically active organic carboxylic acid)] ; and
separating the organic carboxylic acid and unneutralized organic carboxylic acid salt obtained in the second neutralization step from each other, thereby isolating the excess of one enantiomer, which is in the unneutralized organic carboxylic acid salt;
wherein the optically active organic carboxylic acid is a chroman carboxylic acid represented by the general formula (1) or 2-(4-isobutylphenyl) propionic acid: 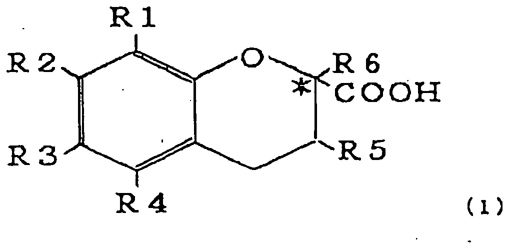 wherein R1, R2, and R4 to R6 each independently represent a methyl group or a hydrogen atom, R3 represents a hydroxy group, a methoxy group, or a hydrogen atom, and
wherein the optically active organic carboxylic acid has an enantiomeric excess of 2 to 98% ee.

2. A method for producing an optically active organic carboxylic acid, comprising isolating an excess of one enantiomer from an enantiomeric mixture of an optically active organic carboxylic acid where enantiomers are present in a non-equimolar ratio,
the method including:
adding an alkali to the enantiomeric mixture of an optically active organic carboxylic acid to prepare a resulting organic carboxylic acid salt and an unneutralized organic carboxylic acid, the alkali being in an amount of 0.9 to 1.1 times the molar number calculated from [the total molar number of the optically active organic carboxylic acid - (twice the molar number of the enantiomer of the optically active organic carboxylic acid being present in a lower molar proportion of enantiomers of the optically active organic carboxylic acid); and
separating the obtained organic carboxylic acid salt and unneutralized organic carboxylic acid from each other, thereby isolating the excess of one enantiomer, which is in the organic carboxylic acid salt;
wherein the optically active organic carboxylic acid is a chroman carboxylic acid represented by the general formula (1) or 2-(4-isobutylphenyl) propionic acid: 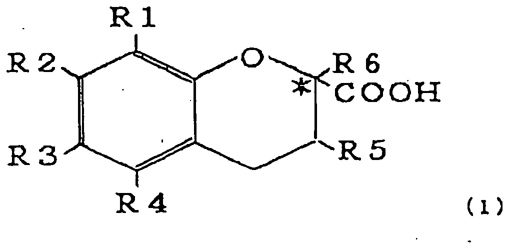 wherein R1, R2, and R4 to R6 each independently represent a methyl group or a hydrogen atom, R3 represents a hydroxy group, a methoxy group, or a hydrogen atom, and
wherein the optically active organic carboxylic acid has an enantiomeric excess of 2 to 98% ee.

3. A method for producing an optically active organic carboxylic acid according to claim 1 or 2, wherein a mixture of equal amounts of enantiomers of the optically active organic carboxylic acid has a melting point higher than the melting points of both the S-form and R-form enantiomers of the optically active organic carboxylic acid.

4. A method for producing an optically active organic carboxylic acid according to any one of claims 1 to 3, wherein the optically active organic carboxylic acid is 6-hydroxy-2,5,7,8-tetramethylchroman-2-carboxylic acid.

5. A method for producing an optically active organic carboxylic acid according to any one of claims 1 to 3, wherein the optically active organic carboxylic acid is 2-(4-isobutylphenyl)propionic acid.

6. A method for producing an optically active organic carboxylic acid according to claim 1, wherein an inorganic acid or an optically inactive organic acid is used as the acid for neutralization.

7. A method for producing an optically active organic carboxylic acid according to claim 1 or 2, wherein a hydroxide or carbonate of an alkali metal, ammonia, or an optically inactive organic amine is used as the alkali for neutralization.

## Patentansprüche

1. Verfahren zur Herstellung einer optisch aktiven organischen Carbonsäure, umfassend Isolieren eines Überschusses eines Enantiomers von einem Enantiomerengemisch einer optisch aktiven organischen Carbonsäure, in welcher Enantiomere in einem nicht-äquimolaren Verhältnis vorhanden sind,
wobei das Verfahren einschließt:
als einen ersten Neutralisationsschritt Neutralisieren der vollständigen Menge des Enantiomerengemisches der optisch aktiven organischen Carbonsäure mit einer Base zum Bereiten eines Gemisches von Enantiomeren eines organischen Carbonsäuresalzes;
als einen zweiten Neutralisationsschritt Hinzufügen einer Säure zu dem Gemisch von Enantiomeren eines organischen Carbonsäuresalzes, erhalten in dem ersten Neutralisationsschritt, zum Bereiten einer daraus entstehenden organischen Carbonsäure und eines nicht-neutralisierten organischen Carbonsäuresalzes, wobei die Säure in einer Menge von 0,9 bis 1,1 mal der Molzahl ist, berechnet aus zweimal der Molzahl des Enantiomers der optisch aktiven organischen Carbonsäure, das in einem geringeren molaren Anteil der Enantiomere der optisch aktiven organischen Carbonsäure vorhanden ist; und
Trennen der organischen Carbonsäure und des nicht-neutralisierten organischen Carbonsäuresalzes, erhalten in dem zweiten Neutralisationsschritt, voneinander, wodurch der Überschuss eines Enantiomers, welches in dem nicht-neutralisierten organischen Carbonsäuresalz ist, isoliert wird;
wobei die optisch aktive organische Carbonsäure eine Chromancarbonsäure, dargestellt durch die allgemeine Formel (1), oder 2-(4-Isobutylphenyl)propionsäure ist: 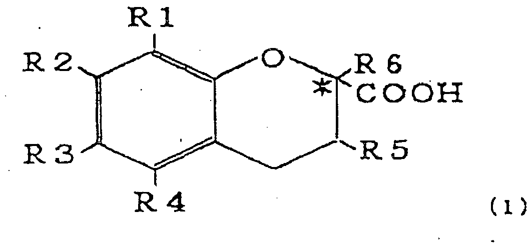
wobei R1, R2 und R4 bis R6 jeweils unabhängig voneinander eine Methylgruppe oder ein Wasserstoffatom darstellen, R3 eine Hydroxygruppe, eine Methoxygruppe oder ein Wasserstoffatom darstellt, und
wobei die optisch aktive organische Carbonsäure einen Enantiomerenüberschuss von 2 bis 98% ee aufweist.

2. Verfahren zur Herstellung einer optisch aktiven organischen Carbonsäure, umfassend Isolieren eines Überschusses eines Enantiomers von einem Enantiomerengemisch einer optisch aktiven organischen Carbonsäure, in welcher Enantiomere in einem nicht-äquimolaren Verhältnis vorhanden sind,
wobei das Verfahren einschließt:
Hinzufügen einer Base zu dem Enantiomerengemisch einer optisch aktiven organischen Carbonsäure zum Bereiten eines daraus entstehenden organischen Carbonsäuresalzes und einer nicht-neutralisierten organischen Carbonsäure, wobei die Base in einer Menge von 0,9 bis 1,1 mal der Molzahl ist, berechnet aus [der Gesamtmolzahl der optisch aktiven organischen Carbonsäure - (zweimal die Molzahl des Enantiomers der optisch aktiven organischen Carbonsäure, das in einem geringeren molaren Anteil der Enantiomere der optisch aktiven organischen Carbonsäure vorhanden ist)]; und
Trennen des erhaltenen organischen Carbonsäuresalzes und der nicht-neutralisierten organischen Carbonsäure voneinander, wodurch der Überschuss eines Enantiomers, welches in dem organischen Carbonsäuresalz ist, isoliert wird;
wobei die optisch aktive organische Carbonsäure eine Chromancarbonsäure, dargestellt durch die allgemeine Formel (1), oder 2-(4-Isobutylphenyl)propionsäure ist: 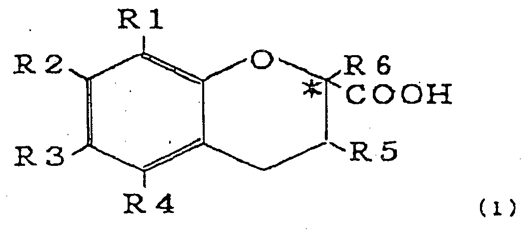
wobei R1, R2 und R4 bis R6 jeweils unabhängig voneinander eine Methylgruppe oder ein Wasserstoffatom darstellen, R3 eine Hydroxygruppe, eine Methoxygruppe oder ein Wasserstoffatom darstellt, und
wobei die optisch aktive organische Carbonsäure einen Enantiomerenüberschuss von 2 bis 98% ee aufweist.

3. Verfahren zur Herstellung einer optisch aktiven organischen Carbonsäure nach Anspruch 1 oder 2, wobei ein Gemisch von gleichen Mengen an Enantiomeren der optisch aktiven organischen Carbonsäure einen höheren Schmelzpunkt aufweist als die Schmelzpunkte beider Enantiomere in S-Konfiguration und R-Konfiguration der optisch aktiven organischen Carbonsäure.

4. Verfahren zur Herstellung einer optisch aktiven organischen Carbonsäure nach einem der Ansprüche 1 bis 3, wobei die optisch aktive organische Carbonsäure 6-Hydroxy-2,5,7,8-tetramethylchroman-2-carbonsäure ist.

5. Verfahren zur Herstellung einer optisch aktiven organischen Carbonsäure nach einem der Ansprüche 1 bis 3, wobei die optisch aktive organische Carbonsäure 2-(4-Isobutylphenyl)propionsäure ist.

6. Verfahren zur Herstellung einer optisch aktiven organischen Carbonsäure nach Anspruch 1, wobei eine anorganische Säure oder eine optisch inaktive organische Säure als die Säure zur Neutralisation verwendet wird.

7. Verfahren zur Herstellung einer optisch aktiven organischen Carbonsäure nach Anspruch 1 oder 2, wobei ein Hydroxid oder ein Carbonat eines Alkalimetalls, Ammoniak oder ein optisch inaktives organisches Amin als die Base zur Neutralisation verwendet wird.

## Revendications

1. Procédé de production d'un acide carboxylique organique optiquement actif, comprenant l'isolement d'un excès d'un énantiomère provenant d'un mélange énantiomérique d'un acide carboxylique organique optiquement actif, dans lequel les énantiomères sont présents selon un rapport non équimolaire,
le procédé comprenant :
en tant que première étape de neutralisation, la neutralisation de la totalité de la quantité du mélange énantiomérique de l'acide carboxylique organique optiquement actif avec un alcalin pour préparer un mélange contenant les énantiomères d'un sel d'acide carboxylique organique ;
en tant que seconde étape de neutralisation, l'ajout d'un acide au mélange contenant les énantiomères d'un sel d'acide carboxylique organique, obtenu dans la première étape de neutralisation, pour préparer un acide carboxylique organique résultant et un sel d'acide carboxylique organique non neutralisé, la quantité d'acide représentant 0,9 à 1,1 fois le nombre de moles calculé à partir de deux fois le nombre de moles de l'énantiomère de l'acide carboxylique organique optiquement actif présent en une proportion molaire plus basse parmi les énantiomères de l'acide carboxylique organique optiquement actif ; et
la séparation entre l'acide carboxylique organique et le sel d'acide carboxylique organique non neutralisé obtenu dans la seconde étape de neutralisation, pour ainsi isoler l'excès d'un énantiomère qui est dans le sel d'acide carboxylique organique non neutralisé ;
dans lequel l'acide carboxylique organique optiquement actif est un acide carboxylique chromanique représenté par la formule générale (1) ou acide 2-(4-isobutylphényl)propionique : 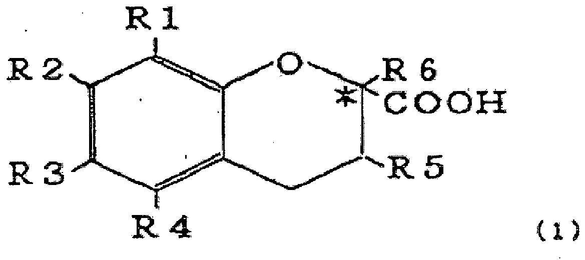
dans laquelle R1, R2 et R4 à R6 représentent chacun indépendamment un groupe méthyle ou un atome d'hydrogène, R3 représente un groupe hydroxy, un groupe méthoxy ou un atome d'hydrogène, et
dans lequel l'acide carboxylique organique optiquement actif présente un excès énantiomérique de 2 % à 98 % ee.

2. Procédé de production d'un acide carboxylique organique optiquement actif, comprenant l'isolement d'un excès d'un énantiomère provenant d'un mélange énantiomérique d'un acide carboxylique organique optiquement actif, dans lequel les énantiomères sont présents selon un rapport non équimolaire,
le procédé comprenant :
l'ajout d'un alcalin au mélange énantiomérique d'un acide carboxylique organique optiquement actif pour préparer un sel d'acide carboxylique organique résultant et un acide carboxylique organique non neutralisé, la quantité d'alcalin représentant 0,9 à 1,1 fois le nombre de moles calculé à partir [du nombre total de mole de l'acide carboxylique organique optiquement actif - (deux fois le nombre de moles de l'énantiomère de l'acide carboxylique organique optiquement actif présent en une proportion molaire plus basse parmi les énantiomères de l'acide carboxylique organique optiquement actif)] ; et
la séparation entre le sel d'acide carboxylique organique obtenu et l'acide carboxylique organique non neutralisé, pour ainsi isoler l'excès d'un énantiomère qui est dans le sel d'acide carboxylique organique ;
dans lequel l'acide carboxylique organique optiquement actif est un acide carboxylique de chromanique représenté par la formule générale (1) ou l'acide 2-(4-isobutylphényl)propionique : 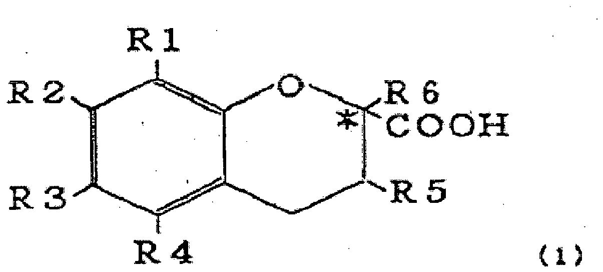
dans laquelle R1, R2 et R4 à R6 représentent chacun indépendamment un groupe méthyle ou un atome d'hydrogène, R3 représente un groupe hydroxy, un groupe méthoxy ou un atome d'hydrogène, et
dans lequel l'acide carboxylique organique optiquement actif présente un excès énantiomérique de 2 % à 98 % ee.

3. Procédé de production d'un acide carboxylique organique optiquement actif selon la revendication 1 ou 2, dans lequel un mélange de quantités égales d'énantiomères de l'acide carboxylique organique optiquement actif possède un point de fusion supérieur aux points de fusion des deux énantiomères de forme S et de forme R de l'acide carboxylique organique optiquement actif.

4. Procédé de production d'un acide carboxylique organique optiquement actif selon l'une quelconque des revendications 1 à 3, dans lequel l'acide carboxylique organique optiquement actif est l'acide 6-hydroxy-2,5,7,8-tétraméthylchroman-2-carboxylique.

5. Procédé de production d'un acide carboxylique organique optiquement actif selon l'une quelconque des revendications 1 à 3, dans lequel l'acide carboxylique organique optiquement actif est l'acide 2-(4-isobutyl-phényl)propionique.

6. Procédé de production d'un acide carboxylique organique optiquement actif selon la revendication 1, dans lequel un acide inorganique ou un acide organique optiquement inactif est utilisé comme acide pour la neutralisation.

7. Procédé de production d'un acide carboxylique organique optiquement actif selon la revendication 1 ou 2, dans lequel un hydroxyde ou un carbonate d'un métal alcalin, l'ammoniac ou une amine organique optiquement inactive est utilisé comme alcalin pour la neutralisation.
